Europäisches Patentamt

European Patent Office

Office européen des brevets

(19)

(11) Numéro de publication: **0 146 474**
A2

(12) # DEMANDE DE BREVET EUROPEEN

(21) Numéro de dépôt: 84402593.2

(22) Date de dépôt: 14.12.84

(51) Int. Cl.⁴: **A 61 K 37/18, A 61 K 9/08**

(30) Priorité: 20.12.83 FR 8320382

(43) Date de publication de la demande: 26.06.85
Bulletin 85/26

(84) Etats contractants désignés: **AT BE CH DE FR GB IT LI LU NL SE**

(71) Demandeur: **SYNTHELABO, 58, rue de la Glacière, F-75621 Paris Cedex 13 (FR)**

(72) Inventeur: **Rose, Francis M., 5, rue des Handriettes, F-75003 Paris (FR)**
Inventeur: **Wessely, Jean-Yves M., 7, Avenue du Bel Air, F-92270 Bois-Colombes (FR)**

(74) Mandataire: **Thouret-Lemaître, Elisabeth et al, Service Brevets - SYNTHELABO 58, rue de la Glacière, F-75621 Paris Cedex 13 (FR)**

(54) Composition nutritive contenant de l'acide alpha-cétoisocaproïque ou un de ses sels.

(57) Soluté pour l'alimentation parentérale, entérale ou orale, caractérisé par le fait qu'il contient de l'acide alpha-cétoisocaproïque ou un ou plusieurs de ses sels.
Application en thérapeutique.

EP 0 146 474 A2

1    0146474

La présente invention a pour objet une composition
nutritive, contenant de l'acide alpha-cétoisocaproïque ou un
de ses sels, destinée à l'alimentation parentérale, entérale
ou orale.

En 1975 Fulks et coll. (Fulks, R.M. J.B. and Goldberg, A.L.,
Effect of insulin, glucose and amino acids on protein
turnover in rat diaphragm, J. Biol. Chem., 250, 290, 1975)
et Buse et coll. (Buse, M.G. and Reid, S.S., Leucine : a
possible regulator of protein turnover, J. Clin. Invest.,
56, 1250, 1975) émettent, à partir d'expériences sur des
cellules isolées de muscle de rat, l'hypothèse d'une action
bivalente de la leucine, capable à la fois d'inhiber la
dégradation des protéines et de favoriser leur synthèse. Par
la suite, il a été montré que l'action sur la protéolyse
était propre à l'acide alpha-cétoisocaproïque, acide
alpha-cétonique analogue de la leucine, tandis que la
leucine, en tant que telle agissait sur la stimulation de la
synthèse des protéines. Plusieurs études biochimiques au
niveau cellulaire ou sur des organes isolés d'animaux ont
permis de mettre en évidence ces actions (Chua, B ; Siehl,
D.L. and Morgan, H.E., Effect of leucine and metabolites of
branched chain amino acids on protein turnover in heart, J.
Biol. Chem., 254, 8358, 1979 ; Tischler, M.E. and Goldberg,
A.L., Effects of alpha-cetoisocaproate on nitrogen
metabolism. Fed. Proc. Fed. Am. Soc. Exp. Biol., 39, 1682,
1980 ; Goldberg A.L. and Tischler, M.E., Regularoty effects
of leucine on carbohydrate and protein metabolism in :
Metabolism and clinical implications of branched chain amino
and ketoacids. Developments in Biochemistry, vol. 18, p.
205, 1981. Elsevier North-Holland ; Li, J.B. and Jefferson,
L.S. Influence of amino acid availability on protein
turnover in perfused skeletal muscle, Biochim. Biochim.
Biophys. Acta, 544, 351, 1978). Chez des patients ayant subi
une intervention chirurgicale abdominale majeure Sapir et
coll. (Sapir, D.G. ; Stewart, P.M. ; Walser, M. ; Moreadith,
C. ; Moyer, E.D. ; lmbembo, A.L. ; Rosenheim, N.B. and
Munoz, S. Effects of alpha-ketoisocaproate and of leucine on
nitrogen metabolism in postoperative patients, Lancet, 8332, 1010, 1983) démontrent que le sel de sodium de l'acide alpha-cétoisocaproïque, administré en perfusion à la dose de 70 mmole/jour, permet d'obtenir une balance azotée moins négative et une excrétion urinaire moindre de la méthyl-3 histidine, ce qui reflète une protéolyse musculaire diminuée. Dès la dose de 11 mmol, cet effet est détecté. Par contre, l'administration de leucine aux mêmes doses chez des patients identiques ne réduit par l'élimination urinaire de la méthyl-3 histidine.

La présente invention a pour objet des compositions destinées à l'alimentation parentérale, entérale ou orale, caractérisées par le fait qu'elles contiennent de l'acide alpha-cétoisocaproïque ou un ou plusieurs de ses sels. Elles contiennent également les substances nutritionnelles habituellement utilisées par la voie orale, entérale ou parentérale (acides aminés, carbohydrates, électrolytes, oligoéléments, vitamines). Les compositions de l'invention sont capables d'inhiber le catabolisme protéique du muscle et de stimuler les synthèses protéiques.

Les compositions de l'invention sont constituées par un soluté d'acides aminés, qui est un mélange d'acides aminés essentiels et non essentiels et contiennent également de l'acide alpha-cétoisocaproïque ou un ou plusieurs de ses sels, plus particulièrement le sel de sodium.

Les compositions de l'invention contiennent pour 1 litre de solution, en plus de l'acide alpha-cétoisocaproïque, éventuellement sous forme de sel, l'ensemble ou une partie des constituants suivants dans les quantités suivantes, données en g :

| | |
|---|---|
| Acide alpha-cétoisocaproïque (éventuellement sous forme de sel) | 4,0 à 16 |
| L-Leucine | 5,0 à 12 |
| L-Isoleucine | 3,0 à 10 |
| L-Valine | 5,0 à 10 |
| L-Lysine | 3,0 à 7 |

| | |
|---|---|
| L-Méthionine | 1,5 à 5 |
| L-Phénylalanine | 1,5 à 7 |
| L-Thréonine | 3,0 à 6 |
| L-Tryptophane | 1,0 à 3 |
| L-Arginine | 5,0 à 10 |
| L-Histidine | 2,5 à 5 |
| L-Alanine | 3,0 à 6 |
| Acide L-aspartique | 1,5 à 4 |
| Acide L-glutamique | 2,0 à 6 |
| L-Glutamine | 2,0 à 6 |
| L-Cystéine | 1,0 à 3 |
| Glycine | 3,0 à 7 |
| L-Proline | 4,0 à 8 |
| L-Sérine | 2,5 à 6 |
| L-Tyrosine | 0,2 à 5 |
| L-Ornithine | 2,0 à 6 |
| Taurine | 1,0 à 4 |
| Eau distillée | q.s.p. 1 l |

La cystéine base ou le chlorhydrate de cystéine peuvent être remplacés par un dérivé tel que la N-acétylcystéine, la N-diacétylcystine ou par un di - ou un tripeptide contenant de la cystéine.

La tyrosine, dont la solubilité est limitée à environ 0,45 g/l, peut être apportée lorsque l'on souhaite des concentrations plus élevées, sous forme de N-acétyl-tyrosine ou d'un di - ou tripeptide contenant de la tyrosine. Ces produits sont en effet bien solubles.

La glutamine, connue pour son instabilité, peut éventuellement être utilisée sous forme de peptides, stables, en solution, ce qui permet un apport de glutamine à l'organisme.

Les exemples suivants illustrent l'invention. 0146474

Exemple 1 :

Acide alpha-cétoisocaproïque (éventuellement

|  | |
|---|---|
| sous forme de sel) | 8,80 g |
| L-Leucine | 8,00 |
| L-Isoleucine | 5,56 |
| L-Valine | 6,05 |
| L-Lysine | 4,80 |
| L-Méthionine | 3,00 |
| L-Phénylalanine | 3,66 |
| L-Thréonine | 2,93 |
| L-Tryptophane | 1,46 |
| L-Arginine | 8,00 |
| L-Histidine | 2,50 |
| L-Alanine | 5,33 |
| Acide L-aspartique | 2,66 |
| Acide L-glutamique | 2,67 |
| N-acétyl-L-Cystéine | 1,80 |
| Glycine | 9,33 |
| L-Proline | 5,33 |
| L-Sérine | 2,66 |
| L-Tyrosine | 0,40 |
| N-acétyl-L-tyrosine | 3,00 |
| L-Ornithine | 2,38 |
| Eau distillée | q.s.p. 1 l |

Exemple 2 :

| | |
|---|---|
| Acide alpha-cétoisocaproïque | 8,80 g |
| L-Leucine | 8,00 |
| L-Isoleucine | 5,56 |
| L-Valine | 6,05 |
| L-Lysine | 4,80 |
| L-Méthionine | 3,00 |
| L-Phénylalanine | 3,66 |
| L-Thréonine | 2,93 |
| L-Tryptophane | 1,46 |

| | |
|---|---|
| L-Arginine | 8,00 |
| L-Histidine | 2,50 |
| L-Alanine | 5,33 |
| Acide L-aspartique | 2,66 |
| Acide L-glutamique | 2,67 |
| Glycyl-L-Glutamine | 8,00 |
| N-Acétyl-L-Cystéine | 1,80 |
| Glycine | 7,00 |
| L-Proline | 5,33 |
| L-Sérine | 2,66 |
| L-Tyrosine | 0,40 |
| N-Acétyl-L-Tyrosine | 3,00 |
| L-Ornithine | 2,38 |
| Eau distillée | q.s.p. 1 l |

Exemple 3 :

| | |
|---|---|
| Alpha-cétoisocaproate de sodium | 10,35 g |
| L-Leucine | 8,00 |
| L-Isoleucine | 5,56 |
| L-Valine | 6,05 |
| L-Lysine | 4,80 |
| L-Méthionine | 3,00 |
| L-Phénylalanine | 3,66 |
| L-Thréonine | 2,93 |
| L-Tryptophane | 1,46 |
| L-Arginine | 8,00 |
| L-Histidine | 2,50 |
| L-Alanine | 5,33 |
| Acide L-aspartique | 2,66 |
| Acide L-glutamique | 2,67 |
| N-acétyl-L-Cystéine | 1,80 |
| Glycine | 9,33 |
| L-Proline | 5,33 |
| L-Sérine | 2,66 |
| L-Tyrosine | 0,40 |
| L-Alanyl-L-Tyrosine | 4,20 |
| L-Ornithine | 2,38 |
| Eau distillée | q.s.p. 1 l |

Exemple 4 :

| | |
|---|---|
| Alpha-cétoisocaproate de sodium | 10,35 g |
| L-Leucine | 8,00 |
| L-Isoleucine | 5,56 |
| L-Valine | 6,05 |
| L-Lysine | 4,80 |
| L-Méthionine | 3,00 |
| L-Phénylalanine | 3,66 |
| L-Thréonine | 2,93 |
| L-Tryptophane | 1,46 |
| L-Arginine | 8,00 |
| L-Histidine | 2,50 |
| L-Alanine | 5,33 |
| Acide L-aspartique | 2,66 |
| Acide L-glutamique | 2,67 |
| N-Acétyl-L-Cystéine | 1,80 |
| Glycine | 9,33 |
| L-Proline | 5,33 |
| L-Sérine | 2,66 |
| L-Tyrosine | 0,40 |
| Glycyl-L-Tyrosine | 4,00 |
| L-Ornithine | 2,38 |
| Eau distillée | q.s.p. 1 l |

Exemple 5 :

| | |
|---|---|
| Acide alpha-cétoisocaproïque | 8,80 g |
| L-Leucine | 8,00 |
| L-Isoleucine | 5,56 |
| L-Valine | 6,05 |
| L-Lysine | 4,80 |
| L-Méthionine | 3,00 |
| L-Phénylalanine | 3,66 |
| L-Thréonine | 2,93 |
| L-Tryptophane | 1,46 |
| L-Arginine | 8,00 |
| L-Histidine | 2,50 |
| L-Alanine | 5,33 |

| | |
|---|---|
| Acide L-aspartique | 2,66 |
| Acide L-glutamique · | 2,67 |
| L-cystinyl-bis-L-alanine | 3,50 |
| Glycine | 9,33 |
| L-Proline | 5,33 |
| L-Sérine | 2,66 |
| L-Tyrosine | 0,40 |
| Gly-L-Tyrosine | 4,00 |
| L-Ornithine | 2,38 |
| Eau distillée | q.s.p. 1 l |

Exemple 6 :

| | |
|---|---|
| Acide alpha-cétoisocaproïque | 8,80 g |
| L-Leucine | 8,00 |
| L-Isoleucine | 5,56 |
| L-Valine | 6,05 |
| L-Lysine | 4,80 |
| L-Méthionine | 3,00 |
| L-Phénylalanine | 3,66 |
| L-Thréonine | 2,93 |
| L-Tryptophane | 1,46 |
| L-Arginine | 8,00 |
| L-Histidine | 2,50 |
| L-Alanine | 5,33 |
| Acide L-aspartique | 2,66 |
| Acide L-glutamique | 2,67 |
| L-alanyl-L-glutamine | 8,00 |
| L-cystinyl-bis-L-alanine | 3,50 |
| Glycine | 9,33 |
| L-Proline | 5,33 |
| L-Sérine | 2,66 |
| L-Tyrosine | 0,40 |
| $N^2$-L-tyrosine-$N^6$-L-tyrosinyl-L-lysine | 4,00 |
| L-Ornithine | 2,38 |
| Eau distillée | q.s.p. 1 l |

Exemple 7 :

| | |
|---|---|
| Alpha-cétoisocaproate de sodium | 10,35 g |
| L-Leucine | 8,00 |
| L-Isoleucine | 5,00 |
| L-Valine | 8,00 |
| L-Lysine | 5,00 |
| L-Méthionine | 3,00 |
| L-Phénylalanine | 2,00 |
| L-Thréonine | 4,00 |
| L-Tryptophane | 1,07 |
| L-Arginine | 6,05 |
| L-Histidine | 3,70 |
| L-Alanine | 3,50 |
| Acide L-aspartique | 3,00 |
| Acide L-glutamique | 3,00 |
| N-acétylcystéine | 1,90 |
| Glycine | 5,00 |
| L-Proline | 7,00 |
| L-Sérine | 4,00 |
| L-Tyrosine | 0,40 |
| N-acétyl-L-tyrosine | 1,40 |
| L-Ornithine (chlorhydrate) | 4,62 |
| Eau distille | q.s.p. 1 l |

Exemple 8 :

| | |
|---|---|
| Acide alpha-cétoisocaproïque | 8,80 g |
| L-Isoleucine | 5,00 |
| L-Valine | 8,00 |
| L-Lysine | 5,00 |
| L-Méthionine | 3,00 |
| L-Phénylalanine | 2,00 |
| L-Thréonine | 4,00 |
| L-Tryptophane | 1,07 |
| L-Arginine | 6,05 |
| L-Histidine | 3,70 |
| L-Alanine | 3,50 |
| Acide L-aspartique | 3,00 |

| | |
|---|---|
| Acide L-glutamique | 3,00 |
| N-acétyl-L-cystéine | 1,90 |
| Glycine | 5,00 |
| L-Proline | 7,00 |
| L-Sérine | 4,00 |
| L-Tyrosine | 0,40 |
| N-acétyl-L-tyrosine | 1,40 |
| L-Ornithine (chlorhydrate) | 4,62 |
| Eau distillée | q.s.p. 1 l |

Exemple 9 :

| | |
|---|---|
| Alpha-cétoisocaproate de sodium | 10,35 g |
| L-Isoleucine | 5,00 |
| L-Valine | 8,00 |
| L-Lysine | 5,00 |
| L-Méthionine | 3,00 |
| L-Phénylalanine | 2,00 |
| L-Thréonine | 4,00 |
| L-Tryptophane | 1,07 |
| L-Arginine | 6,05 |
| L-Histidine | 3,70 |
| L-Alanine | 3,50 |
| Acide L-aspartique | 3,00 |
| Glycyl-L-glutamine | 8,00 |
| Acide L-glutamique | 3,00 |
| N-acétyl-L-cystéine | 1,90 |
| Glycine | 5,00 |
| L-Proline | 7,00 |
| L-Sérine | 4,00 |
| L-Tyrosine | 0,40 |
| N-acétyl-L-tyrosine | 1,40 |
| L-Ornithine (chlorhydrate) | 4,62 |
| Eau distillée | q.s.p. 1 l |

Les solutés obtenus selon l'invention peuvent être stérilisés soit par un procédé de filtration stérilisante, soit par la chaleur.

A ces solutés d'acides aminés, on peut ajouter du glucose ou un autre carbohydrate utilisé en alimentation parentérale (fructose, sorbitol, maltose, etc...), une émulsion lipidique préparée à base de toute huile utilisable par voie veineuse (huile des soja, de carthame, etc, et/ou des triglycérides à chaîne moyenne) et d'un émulsifiant compatible (lécithine de soja, d'oeuf, etc). Des électrolytes, des oligoéléments, des vitamines peuvent également être ajoutés.

Si l'on utilise les compositions de l'invention pour l'alimentation entérale, on peut ajouter aux mélanges cités des polypeptides, des protéines, des oses, des oligosaccharides et/ou des triglycérides à chaîne longue et à chaîne moyenne.

Les solutés d'acides aminés de l'invention ont pour but de :
- réduire le catabolisme protéique
- et stimuler les synthèses protéiques.

Ils peuvent être utilisés dans les situations métaboliques induisant de telles modifications, généralement regroupées sous la terminologie de stress.

Ils peuvent être utilisés pour l'alimentation de malades
- en phase post-opératoire des interventions chirurgicales
- polytraumatisés et traumatisés du cerveau
- atteints de brûlures graves et étendues
- ayant des complications infectieuses de la chirurgie ou des maladies infectieuses graves,
- en phase aiguë de l'insuffisance rénale aiguë.
- en phase aiguë des maladies inflammatoires de l'intestin.

Les compositions de l'invention peuvent être utilisées chez l'adulte et chez l'enfant.

Les posologies seront adaptées aux besoins spécifiques du patient, besoins qui sont mesurés par les paramètres généralement acceptés, tels que bilan journalier d'azote, mesure de l'élimination urinaire de la méthyl-3 histidine, mesure des protéines sanguines. Ainsi, selon les cas, la quantité administrée peut aller de 500 ml à 2 l de soluté par jour.

0146474

Revendications pour les états contractants : BE, CH, DE, FR, BG, IT, LI, LU, NL, SE.

1. Soluté pour l'alimentation parentérale, entérale ou orale, caractérisé par le fait qu'il contient de l'acide alpha-cétoisocaproïque ou un ou plusieurs de ses sels.

2. Soluté pour l'alimentation parentérale, entérale ou orale selon la revendication 1, caractérisé par le fait qu'il contient des acides aminés essentiels et non essentiels.

3. Soluté pour l'alimentation parentérale, entérale ou orale, selon la revendication 1 ou la revendication 2, caractérisé par le fait que certains des acides aminés sont sous la forme de dérivés (sels ou dérivés N-acétylés ou peptides).

4. Soluté selon l'une quelconque des revendications 1, 2 et 3, caractérisé par le fait qu'il contient également des hydrates de carbone et/ou des lipides et/ou des électrolytes et/ou des oligoéléments et/ou des vitamines.

5. Soluté selon l'une quelconque des revendicaitons 1 à 4, caractérisé par le fait qu'il contient de la leucine.

6. Soluté selon l'une quelconque des revendications 1 à 4, caractérisé par le fait qu'il ne contient pas de leucine.

7. Soluté selon l'une quelconque des revendications 1 à 5, caractérisé par le fait qu'il a la composition suivante

| | |
|---|---|
| Acide alpha-cétoisocaproïque (éventuellement sous forme de sel) | 4,0 à 16g |
| L-Leucine | 5,0 à 12 |
| L-Isoleucine | 3,0 à 10 |
| L-Valine | 5,0 à 10 |
| L-Lysine | 3,0 à 7 |
| L-Méthionine | 1,5 à 5 |
| L-Phénylalanine | 1,5 à 7 |

| | |
|---|---|
| L-Thréonine | 3,0 à 6 |
| L-Tryptophane | 1,0 à 3 |
| L-Arginine | 5,0 à 10 |
| L-Histidine | 2,5 à 5 |
| L-Alanine | 3,0 à 6 |
| Acide L-aspartique | 1,5 à 4 |
| Acide L-glutamique | 2,0 à 6 |
| L-Glutamine | 2,0 à 6 |
| L-Cystéine | 1,0 à 3 |
| Glycine | 3,0 à 7 |
| L-Proline | 4,0 à 8 |
| L-Sérine | 2,5 à 6 |
| L-Tyrosine | 0,2 à 5 |
| L-Ornithine | 2,0 à 6 |
| Taurine | 1,0 à 4 |
| Eau distillée | q.s.p. 1 l |

8. Soluté selon l'une quelconque des revendications 1 à 5, caractérisé par le fait qu'il a la composition suivante :

| | |
|---|---|
| Acide alpha-cétoisocaproïque | 8,80 g |
| L-Leucine | 8,00 |
| L-Isoleucine | 5,00 |
| L-Valine | 8,00 |
| L-Lysine | 5,00 |
| L-Méthionine | 3,00 |
| L-Phénylalanine | 2,00 |
| L-Thréonine | 4,00 |
| L-Tryptophane | 1,07 |
| L-Arginine | 6,05 |
| L-Histidine | 3,70 |
| L-Alanine | 3,50 |
| Acide L-aspartique | 3,00 |
| Acide L-glutamique | 3,00 |
| N-acétyl-L-cystéine | 1,90 |
| Glycine | 5,00 |
| L-Proline | 7,00 |
| L-Sérine | 4,00 |
| L-Tyrosine | 0,40 |
| N-acétyl-L-tyrosine | 1,40 |

13

L-Ornithine (chlorhydrate)                              4,62
Eau distillée                                           q.s.p. 1 l

9. Soluté selon l'une quelconque des revendications 1 à 4 et 6, caractérisé par le fait qu'il a la composition suivante :

| | |
|---|---|
| Alpha-cétoisocaproate de sodium | 10,35 g |
| L-Isoleucine | 5,00 |
| L-Valine | 8,00 |
| L-Lysine | 5,00 |
| L-Méthionine | 3,00 |
| L-Phénylalanine | 2,00 |
| L-Thréonine | 4,00 |
| L-Tryptophane | 1,07 |
| L-Arginine | 6,05 |
| L-Histidine | 3,70 |
| L-Alanine | 3,50 |
| Acide L-aspartique | 3,00 |
| Glycyl-L-glutamine | 8,00 |
| Acide L-glutamique | 3,00 |
| N-acétyl-L-cystéine | 1,90 |
| Glycine | 5,00 |
| L-Proline | 7,00 |
| L-Sérine | 4,00 |
| L-Tyrosine | 0,40 |
| N-acétyl-L-tyrosine | 1,40 |
| L-Ornithine (chlorhydrate) | 4,62 |
| Eau distillée | q.s.p. 1 l |

10. Médicament utile pour le traitement de malades en état catabolique, caractérisé par le fait qu'il est constitué par un soluté tel que spécifié dans l'une quelconque des revendications 1 à 9.

Revendications pour l'état contractant 0:146474

1. Procédé dé préparation d'un soluté pour l'alimentation parentérale, entérale ou orale, caractérisé par le fait qu'on ajoute au soluté de l'acide alpha-cétoisocaproïque ou un ou plusieurs de ses sels.

2. Procédé selon la revendication 1, caractérisé par le fait que le soluté contient des acides aminés essentiels et non essentiels.

3. Procédé selon la revendication 1 ou la revendication 2, caractérisé par le fait que certains des acides aminés sont sous la forme de dérivés (sels ou dérivés N-acétylés ou peptides).

4. Procédé selon l'une quelconque des revendications 1, 2 et 3, caractérisé par le fait que le soluté contient également des hydrates de carbone et/ou des lipides et/ou des électrolytes et/ou des oligoéléments et/ou des vitamines.

5. Procédé selon l'une quelconque des revendicaitons 1 à 4, caractérisé par le fait que le soluté contient de la leucine.

6. Procédé selon l'une quelconque des revendications 1 à 4, caractérisé par le fait que le soluté ne contient pas de leucine.

7. Procédé selon l'une quelconque des revendications 1 à 5, caractérisé par le fait que l'on prépare le soluté en utilisant les proportions suivantes

| Acide alpha-cétoisocaproïque (éventuellement sous forme de sel) | 4,0 à 16g |
|---|---|
| L-Leucine | 5,0 à 12 |
| L-Isoleucine | 3,0 à 10 |
| L-Valine | 5,0 à 10 |
| L-Lysine | 3,0 à 7 |

| | |
|---|---|
| L-Méthionine | |
| L-Phénylalanine | 1,5 à 7 |
| L-Thréonine | 3,0 à 6 |
| L-Tryptophane | 1,0 à 3 |
| L-Arginine | 5,0 à 10 |
| L-Histidine | 2,5 à 5 |
| L-Alanine | 3,0 à 6 |
| Acide L-aspartique | 1,5 à 4 |
| Acide L-glutamique | 2,0 à 6 |
| L-Glutamine | 2,0 à 6 |
| L-Cystéine | 1,0 à 3 |
| Glycine | 3,0 à 7 |
| L-Proline | 4,0 à 8 |
| L-Sérine | 2,5 à 6 |
| L-Tyrosine | 0,2 à 5 |
| L-Ornithine | 2,0 à 6 |
| Taurine | 1,0 à 4 |
| Eau distillée | q.s.p. 1 l |

8. Procédé selon l'une quelconque des revendications 1 à 5, caractérisé par le fait que les proportions sont les suivantes :

| | |
|---|---|
| Acide alpha-cétoisocaproïque | 8,80 g |
| L-Leucine | 8,00 |
| L-Isoleucine | 5,00 |
| L-Valine | 8,00 |
| L-Lysine | 5,00 |
| L-Méthionine | 3,00 |
| L-Phénylalanine | 2,00 |
| L-Thréonine | 4,00 |
| L-Tryptophane | 1,07 |
| L-Arginine | 6,05 |
| L-Histidine | 3,70 |
| L-Alanine | 3,50 |
| Acide L-aspartique | 3,00 |
| Acide L-glutamique | 3,00 |
| N-acétyl-L-cystéine | 1,90 |
| Glycine | 5,00 |
| L-Proline | 7,00 |

| | |
|---|---|
| L-Sérine | 4,00 |
| L-Tyrosine | 0,40 |
| N-acétyl-L-tyrosine | 1,40 |
| L-Ornithine (chlorhydrate) | 4,62 |
| Eau distillée | q.s.p. 1 l |

9. Procédé selon l'une quelconque des revendications 1 à 4 et 6, caractérisé par le fait que les proportions sont les suivantes :

| | |
|---|---|
| Alpha-cétoisocaproate de sodium | 10,35 g |
| L-Isoleucine | 5,00 |
| L-Valine | 8,00 |
| L-Lysine | 5,00 |
| L-Méthionine | 3,00 |
| L-Phénylalanine | 2,00 |
| L-Thréonine | 4,00 |
| L-Tryptophane | 1,07 |
| L-Arginine | 6,05 |
| L-Histidine | 3,70 |
| L-Alanine | 3,50 |
| Acide L-aspartique | 3,00 |
| Glycyl-L-glutamine | 8,00 |
| Acide L-glutamique | 3,00 |
| N-acétyl-L-cystéine | 1,90 |
| Glycine | 5,00 |
| L-Proline | 7,00 |
| L-Sérine | 4,00 |
| L-Tyrosine | 0,40 |
| N-acétyl-L-tyrosine | 1,40 |
| L-Ornithine (chlorhydrate) | 4,62 |
| Eau distillée | q.s.p. 1 l |